# EUROPEAN PATENT APPLICATION

(11) **EP 4 545 038 A1**
(43) Date of publication of application: **30.04.2025**
(21) Application number: 23205136.7
(22) Date of filing: 23.10.2023
(51) Int. Cl.: A61B 50/30, A61B 90/00

(54) **A KIT OF MEDICAL DEVICES**

(71) Applicant: Mölnlycke Health Care AB, 431 21 Mölndal (SE)
(72) Inventor: HESTNER, Anders, 435 41 Mölnlycke (SE)
(74) Representative: Kransell & Wennborg KB

(57) **Abstract**

The present disclosure generally relates to a kit of medical devices (1) for a medical intervention, wherein the kit (1) comprises at least a first package (2) comprising one or more medical devices, and a second package (3) comprising one or more medical devices; the medical device(s) of the at least first (2) and second (3) packages being configured for use with the medical intervention, wherein the individual medical device(s) comprised in the first package (2) have a first shelf life which is substantially the same for each component in the first package (2); the individual medical device(s) of the second package (3) having a second shelf life which is substantially the same for each component in the second package (3), and wherein the first shelf life is different from the second shelf life.

The present disclosure also relates to a method for providing a kit of medical devices.

## Description

### TECHNICAL FIELD

The present disclosure generally relates to a kit of medical devices for a medical intervention, wherein the kit comprises at least a first package comprising one or more medical devices and a second package comprising one or more medical devices. The present disclosure also relates to a method for providing such a kit of medical devices.

### BACKGROUND

A medical intervention, such as a surgical procedure, generally requires the use of a plurality of tools and medical devices. In order to reduce the preparation time for the medical personnel prior to a medical intervention, the individual tools and medical devices are often packaged together. Hence, instead of seeking for the individual medical devices required for a specific medical intervention, each component or device that is needed is prearranged in one single package.

For example, one package may contain all the tools and medical devices associated with a hernia repair, a laparoscopic surgery, or a negative pressure wound therapy (NPWT), respectively.

The provision of all the medical devices needed for a specific medical intervention in one single package improves the efficiency and saves considerable time for the medical personnel prior to and during e.g. a surgical procedure.

However, various factors affect how long after manufacturing a component or medical device will maintain the ability to fully perform its intended function.

The shelf life of a medical device is the period during which the device remains suitable for the intended use. The expiry date is the termination of the shelf life, after which the medical device no longer functions as intended.

Various factors affect the shelf life (and expiry date) of a medical device. For example, a medical device may contain substances that degrade over time, or components that interact such that the performance and/or the safety of the medical device is affected. Furthermore, the manufacturing process may alter the chemistry of the raw materials and components of the medical device in an adverse manner. It is also conceivable that the appearance, viscosity, tensile strength, or other physical characteristics of the medical device varies with time.

Storage conditions, sterility, and a number of additional factors also affect the shelf life of a medical device.

One problem associated with providing a plurality of medical devices in one single package is that the individual shelf life of the devices comprised in one package generally differs. Since safety is key in these procedures, the medical device that has the shortest shelf life sets the expiry date for all medical devices in the package.

Hence, after termination of the expiry date, all medical devices in the package are discarded. The result of this is that a plurality of fully functioning medical devices are being discarded too early. This is costly and unsustainable.

Consequently, there is a need to alleviate the problems mentioned hereinbefore and to provide a sustainable and cost-efficient means to improve and facilitate the preparation procedure prior to and during a medical intervention for the medical personnel.

### SUMMARY

In view of the above-mentioned problems, it is an object of the present disclosure to provide improvements with respect to providing a sustainable and cost-efficient means to relieve the burden for the medical personnel prior to and during a medical intervention.

According to a first aspect, there is provided a kit of medical devices for a medical intervention, wherein the kit comprises at least a first package comprising one or more medical devices, and a second package comprising one or more medical devices; the medical device(s) of the at least first and second packages being configured for use with the medical intervention, wherein the individual medical device(s) comprised in the first package have a first shelf life which is substantially the same for each component in the first package; the individual medical device(s) of the second package having a second shelf life which is substantially the same for each component in the second package, and wherein the first shelf life is different from the second shelf life.

With a kit of medical devices according to the present disclosure, the plurality of medical devices associated with a specific medical intervention are arranged in separate packages, and the medical devices are packaged based on their individual shelf life. The components of the first and second packages are configured for use with the same medical intervention.

By arranging the medical devices based on their shelf life, the devices will maintain their intended function for a prolonged period. The scenario of discarding medical devices, such as expensive surgical tools or wound dressings before their actual (individual) expiry date is thereby avoided. Furthermore, the medical devices used by the medical personnel in a specific care scenario are yet arranged in a manner which facilitates the preparation procedure before a specific medical intervention.

The provision of a kit of medical devices packaged separately based on the shelf life is also beneficial since many surgical packages tend to be large and bulky, making it difficult to store and to transport a surgical package from a storage room to an operating room.

Accordingly, a more sustainable and cost-efficient kit of medical devices is provided.

The first shelf life may differ with less than 4 months, preferably less than 3 months between the individual medical device(s) of the first package, and wherein the second shelf life may differ with less than 4 months, preferably less than 3 months between the individual medical device(s) of the second package.

For example, the first shelf life may differ from the second shelf life with at least 6 months.

The shelf life may vary significantly between the individual medical devices used for a specific medical intervention. If all devices were to be packaged in the same package, the medical device having the earliest expiry date, i.e. the shortest shelf life, would set the expiry date for each medical device comprised in that package. However, the provision of a plurality of medical devices in at least a first and a second package, differing in shelf life with at least 6 months, secures that each medical device belonging to the kit can be used for a prolonged period.

The first package may comprise a first expiration marking indicating a first expiry date of the medical device(s) of the first package, and wherein the second package may comprise a second expiration marking indicating a second expiry date of the medical device(s) of the second package, wherein the first expiry date is different from the second expiry date.

The first expiry date corresponds to the earliest expiry date of the medical devices arranged in the first package (i.e. the medical device of the first package having the shortest shelf life).

The second expiry date corresponds to the earliest expiry date of the medical devices arranged in the second package (i.e. the medical device of the first package having the shortest shelf life).

Typically, the medical devices of the first package have substantially the same first expiry date. Likewise, the medical devices of the second package have substantially the same second expiry date.

After completion of the medical intervention, the remaining medical devices contained in the at least first and second packages may be stored and used in future medical interventions (until the end of the first, and second expiry date, respectively).

The second expiry date may be earlier than the first expiry date. For example, the second expiry date may be at least 6 months, e.g. at least 12 months earlier than the first expiry date.

The first and second expiration markings may be in the form of alphanumeric characters, machine readable codes and/or chips.

In exemplary embodiments, the at least first and second packages each comprises at least one kit marking indicating that the first and second packages belong to the same kit for use with the medical intervention.

The provision of kit markings that indicate that the first and second, and optionally additional packages, belong to the same kit significantly facilitates the selection of medical devices for use with a specific medical intervention.

The at least one kit marking may be a graphical element and/or a text element.

For example, the graphical element may be a symbol, a figure, an image, a photograph or a visual element. The graphical element may convey product-identifying information, medical intervention-identifying information, kit-identifying information and/or usage-specific information.

The text element may be a product descriptor, a kit descriptor and/or a medical intervention descriptor.

In exemplary embodiments, the first and second packages may each comprise a color-coded portion; the color-coded portion being indicative of a specific medical intervention.

The color-coded portion facilitates the identification and selection of the components and packages to be used for a specific medical intervention. It is consequently clear to the personnel which packages belong to the same kit and with the same medical intervention, and the various kits and packages can be clearly distinguished from other kits for use with different medical intervention.

The medical intervention may be negative pressure wound therapy (NPWT) or a surgical procedure, for example a laparoscopic procedure or an orthopedic surgical procedure.

Negative pressure wound therapy (NPWT) is a technique that promotes healing of e.g. surgical, acute and chronic wounds by the application of a sub-atmospheric pressure to the wound, using a negative pressure pump. Wound healing is achieved by applying a negative pressure, such as vacuum through a dressing applied onto the wound. Excess wound exudate is thereby drawn out, which increases the blood flow to the area, and promotes the formation of granulation tissue.

In embodiments where the medical intervention is negative pressure wound therapy (NPWT), the kit of medical devices may comprise at least an NPWT device comprising a negative pressure source, and optionally a canister. The kit may also comprise one or a plurality of wound dressing(s), batteries, various tubing assemblies for connecting the dressing with the negative pressure source, adhesive strips to enhance the adherence of the wound dressing to the skin etc.

In a surgical procedure, a vast number of medical devices and components are utilized, including e.g. surgical drapes, gowns, towels, gauzes, tubings, syringes, trocars, scissors, wound dressings etc.

The at least first and second packages may be contained in an outer package.

Hence, the outer package may house the at least first and second packages such that the kit of medical devices is contained in one single outer package.

This may be beneficial to facilitate the preparation procedure for the medical personnel.

According to another aspect, there is provided a method for providing a kit of medical devices for a medical intervention comprising:
- packaging one or more medical devices for the medical intervention in a first package such that each medical device in the first package has substantially the same first shelf life,
- packaging one or more medical devices for the medical intervention in at least a second package such that each medical device in the second package has substantially the same second shelf life, wherein the first shelf life is different from the second shelf life.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 schematically illustrates a kit of medical devices comprising a first package and a second package according to an exemplary embodiment of the present disclosure.
Figure 2 schematically illustrates a plurality of medical devices utilized for negative pressure wound therapy (NPWT).
Figure 3a schematically illustrates a first package comprising a plurality of medical devices for negative pressure wound therapy (NPWT).
Figure 3b schematically illustrates the interior of the first package in figure 3a.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

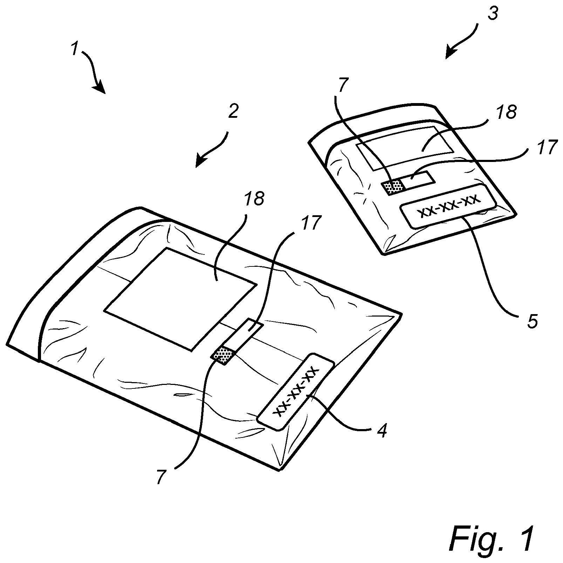

In figure 1, a kit of medical devices 1 according to the present disclosure is schematically illustrated. The kit 1 comprises at least a first package 2 comprising one or more medical devices, and a second package 3 comprising one or more medical devices; the medical device(s) of the first 2 and second 3 packages being configured for use with the medical intervention, wherein the individual medical device(s) comprised in the first package 2 have a first shelf life which is substantially the same for each component in the first package 2; the individual medical device(s) of the second package 3 having a second shelf life which is substantially the same for each component in the second package 3; and wherein the first shelf life is different from the second shelf life.

As used herein, the term "medical device" means any instrument, tool, apparatus, reagent, software, wound dressing or component to be used for a specific medical intervention.

The term "kit of medical devices" means a kit comprising a plurality of medical devices, such as surgical tools, wound dressings, and wound appliances. The kit of medical devices may contain various additional components which do not fall under the category "medical device".

The medical devices are contained in separate packages; i.e. in at least a first and a second package based on the shelf life of the individual medical devices.

The medical devices of the first package are typically different from the medical devices of the second package. Alternatively the medical devices of the first and second packages are the same.

The term "medical intervention" means any measure taken to prevent or treat a disease or condition for the purpose of improving the health of a patient. The medical intervention may be negative pressure wound therapy (NPWT), a surgical procedure, e.g. a laparoscopic procedure or an orthopedic surgical procedure.

The term "shelf life" means the time period that the medical device may be stored without becoming unfit for use, consumption or sale.

The "expiry date" is the termination of the shelf life, after which the medical device no longer is fit for use, consumption, or sale.

The first shelf life is substantially the same for each component in the first package 2, and the second shelf life is substantially the same for each component in the second package 3.

In other words, the first shelf life may differ with less than 4 months, preferably less than 3 months between the individual medical device(s) of the first package 2, and wherein the second shelf life differs with less than 4 months, preferably less than 3 months between the individual medical device(s) of the second package 3.

For example, the shelf life may differ from the second shelf life with at least 6 months.

As illustrated in figure 1, the first package 2 may comprise a first expiration marking 4 indicating a first expiry date of the medical devices of the first package 2, and wherein the second package 3 may comprise a second expiration marking 5 indicating a second expiry date of the medical devices of the second package 3, wherein the first expiry date is different from the second expiry date.

The "first expiry date" corresponds to the expiry date of the component in the first package having the shortest shelf life.

The "second expiry date" corresponds to the expiry date of the component in the second package having the shortest shelf life.

Typically, however, the first expiry date is substantially the same for the components of the first package. Likewise, the second expiry date is substantially the same for the components of the second package.

For example, the first expiry date may differ with less than 4 months, preferably less than 3 months between the individual medical device(s) of the first package 2, and wherein the second expiry date may differ with less than 4 months, preferably less than 3 months between the individual medical device(s) of the second package 3.

The first 4 and second 5 expiration markings may be in the form of alphanumeric characters, machine readable codes and/or chips.

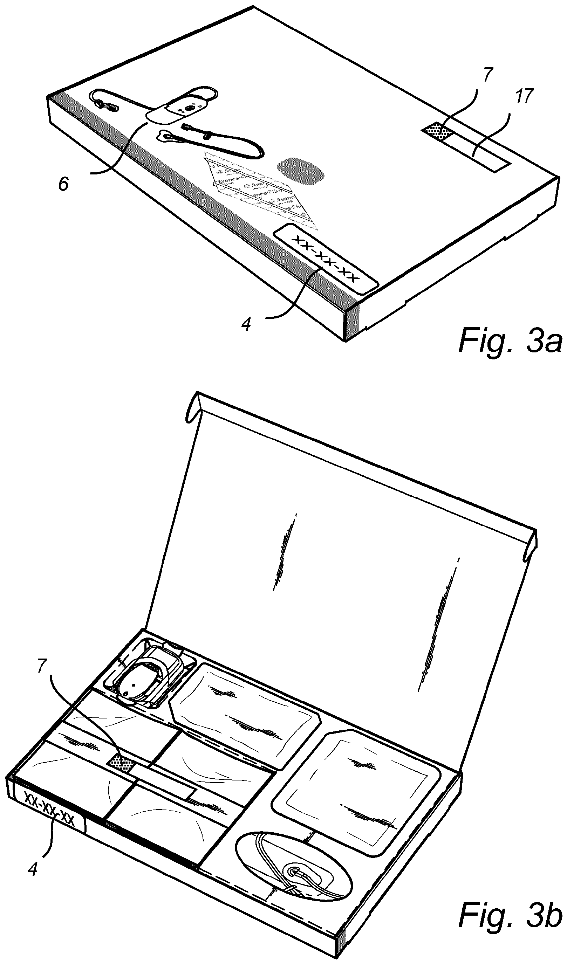

As best illustrated in figure 3, the first 2 and second packages may each comprise at least one kit marking 6 indicating that the first 2 and second 3 packages belong to the same kit for use with the medical intervention.

In figures 3a and 3b, only one of the packages of the kit is shown.

The kit marking is a printed marking.

The kit marking facilitates the selection of packages containing the necessary components and medical devices for a specific medical intervention.

The at least one kit marking 6 may be a graphical element and/or a text element.

As used herein, the term "graphical element" means a symbol, a figure, an image, a photograph, a logotype, a shape, a pattern, or a visual element. The graphical element may be used to convey information, e.g. product-identifying information, medical intervention-identifying information and/or usage-specific information.

In figure 3, the kit markings 6 are graphical elements in the form of product-identifying images.

The "text element" may be a product descriptor, a kit descriptor or a medical intervention descriptor.

The text element may be provided in the tag 17 in figure 1 and in figure 3a.

For example, the text element may be "Laparoscopy" or "Minimal Invasive Surgery" in figure 1. The text element may be "Negative Pressure Wound Therapy" in figure 3a.

As illustrated in figure 1, the outer layer of the first and second packages may contain a list of medical devices comprised in the first 2, and second 3 package, respectively (see box 18 in figure 1).

The first 2 and second 3 packages may each comprise a color-coded portion 7; the color-coded portion 7 being indicative of a specific medical intervention.

The color-coded portion 7 facilitates the identification and selection of the components and packages to be used for a specific medical intervention. Furthermore, the various kits and packages can be clearly distinguished from other kits for use with different medical interventions.

As mentioned hereinbefore, the medical intervention may be negative pressure wound therapy (NPWT), a surgical procedure, e.g. a laparoscopic surgical procedure or an orthopedic surgical procedure.

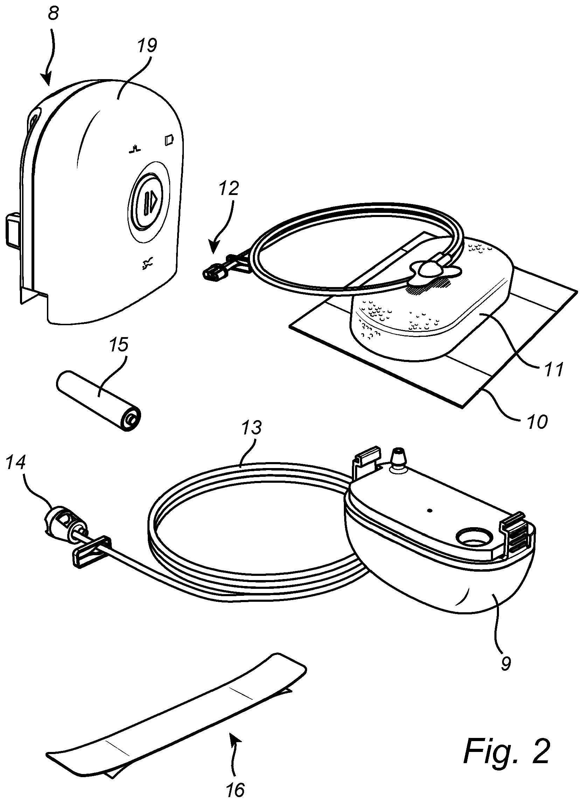

In figure 2, a plurality of components and medical devices used for negative pressure wound therapy (NPWT) are schematically illustrated.

In the context of the present disclosure, "negative pressure wound therapy" refers to a therapy utilizing a source of negative pressure (e.g. a vacuum pump) to remove excess fluid from a wound. The wound may be an open wound or it may be a closed wound; i.e. a surgically closed incision, and the term therefore also encompasses "topical negative pressure (TNP) therapy" applications, which is a term often used in the context of closed incisions.

As illustrated in figure 2, the kit of medical devices may comprise an NPWT device 8 comprising a negative pressure pump. The NPWT device 8 may comprise a control unit electrically connected to the battery and the negative pressure pump. The control unit may be adapted to ensure that the negative pressure provided is within a predetermined range.

The NPWT device 8 may comprise a canister 9. The NPWT device 8 may comprise a housing 19 in which the negative pressure pump is arranged. The canister 9, if present, may be detachably connected to the housing. The kit may further comprise at least one wound dressing 10, which may be absorbent or non-absorbent. In cases where the dressing 10 is non-absorbent, the kit may further comprise a wound filler material 11, such as a gauze or a foam 11.

The kit may further comprise a tubing assembly configured to fluidly connect the wound dressing 10 to the NPWT device 8. The tubing assembly may comprise a first tubing 12 configured to be connected to the dressing 10, and a second tubing 13 configured to be connected to the canister 9.

The kit may also comprise a connector unit 14 attached (detachably or firmly) to the first 12 or second tubing 13 of the tubing assembly. The connector unit 14 may comprise an air filter configured to supply air to the dressing during use. In use, the connector unit 14 is arranged at a position between the wound dressing 10 and the NPWT device 8.

The kit may further comprise batteries 15 for the negative pressure pump, and adhesive strips 16 for enhancing the attachment of the wound dressing 10 onto the skin during use.

At least some of the medical devices illustrated in figure 2 may be contained in a first package of the kit, as e.g. illustrated in figure 3a and 3b.

The present disclosure is not limited to a specific medical intervention, but any medical intervention requiring a plurality of medical devices is conceivable.

The at least first 2 and second 3 packages are not limited to a specific material, shape or configuration. The packages may be in the form of surgical packs as illustrated in figure 1 or as cartons or paper boxes as illustrated in figure 3a and 3b.

It is also conceivable that the first 2 and second 3 packages are contained in the same outer package.

The outer package may house the at least first and second packages, but the packages will contain a respective shelf life (and expiry date) being specific for that package.

In another aspect, there is provided a method for providing a kit of medical devices 1 for a medical intervention comprising:
- packaging one or more medical devices for the medical intervention in a first package 2 such that each medical device in the first package 2 has substantially the same first shelf life,
- packaging one or more medical devices for the medical intervention in at least a second package 3 such that each medical device in the second package 3 has substantially the same second shelf life, wherein the first shelf life is different from the second shelf life.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A kit of medical devices (1) for a medical intervention, wherein said kit (1) comprises at least a first package (2) comprising one or more medical devices, and a second package (3) comprising one or more medical devices; said medical device(s) of said at least first (2) and second (3) packages being configured for use with said medical intervention, wherein the individual medical device(s) comprised in said first package (2) have a first shelf life which is substantially the same for each component in said first package (2); the individual medical device(s) of said second package (3) having a second shelf life which is substantially the same for each component in said second package (3), and wherein said first shelf life is different from said second shelf life.

2. The kit of medical devices (1) according to claim 1, wherein said first shelf life differs with less than 4 months, preferably less than 3 months, between the individual medical device(s) of said first package (2), and wherein said second shelf life differs with less than 4 months, preferably less than 3 months, between the individual medical device(s) of said second package (3).

3. The kit of medical devices (1) according to claim 1 or claim 2, wherein said first shelf life differs from said second shelf life with at least 6 months.

4. The kit of medical devices (1) according any one of the preceding claims, wherein said first package (2) comprises a first expiration marking (4) indicating a first expiry date of the medical device(s) of said first package (2), and wherein said second package (3) comprises a second expiration marking (5) indicating a second expiry date of the medical device(s) of said second package (3), wherein said first expiry date is different from said second expiry date.

5. The kit of medical devices (1) according to claim 4, wherein said first (4) and second (5) expiration markings are in the form of alphanumeric characters, machine readable codes and/or chips.

6. The kit of medical devices (1) according to any one of the preceding claims, wherein said at least first (2) and second (3) packages each comprises at least one kit marking (6) indicating that said first (2) and second (3) packages belong to the same kit for use with said medical intervention.

7. The kit of medical devices (1) according to claim 6, wherein said at least one kit marking is a graphical element and/or a text element.

8. The kit of medical devices (1) according to claim 6 or claim 7, wherein said first (2) and second (3) packages each comprises a color-coded portion (7); said color-coded portion (7) being indicative of a specific medical intervention.

9. The kit of medical devices (1) according to any one of the preceding claims, wherein said medical intervention is negative pressure wound therapy (NPWT) or a surgical procedure, for example a laparoscopic procedure or an orthopedic surgical procedure.

10. The kit of medical devices (1) according to any one of the preceding claims, wherein said at least first (2) and second (3) packages are contained in an outer package.

11. A method for providing a kit of medical devices (1) for a medical intervention comprising
- packaging one or more medical devices for said medical intervention in a first package (2) such that each medical device in said first package (2) has substantially the same first shelf life,
- packaging one or more medical devices for said medical intervention in at least a second package (3) such that each medical device in said second package (3) has substantially the same second shelf life, wherein said first shelf life is different from said second shelf life.
